# EUROPEAN PATENT SPECIFICATION

(11) **EP 3 398 641 B1**
(45) Date of publication and mention of the grant of the patent: **24.06.2020**
(21) Application number: 18180731.4
(22) Date of filing: 28.05.2014
(51) Int. Cl.: A61M 16/10, A61M 11/00, A61M 16/14, C25B 1/04, C25B 1/00, A61M 15/00, C25B 15/08, C25B 9/00, A61M 16/00

(54) **ANTI-EXPLOSION GAS GENERATOR FOR HEALTH USE**
EXPLOSIONSGESCHÜTZTER GASGENERATOR ZUR GESUNDHEITSVERWENDUNG
GÉNÉRATEUR DE GAZ ANTI-EXPLOSION POUR UTILISATION MÉDICALE

(30) Priority: 19.06.2013 CN 201320352900 U
(43) Date of publication of application: 07.11.2018
(62) Divisional of application: 14170279.5
(73) Proprietor: Lin, Hsin-Yung, Shanghai 201-801 (CN)
(72) Inventor: Lin, Hsin-Yung, Shanghai 201-801 (CN)
(74) Representative: TBK

(56) References cited:
- EP-A1- 2 484 811
- CN-U- 202 576 577
- US-A- 5 672 581
- US-A1- 2010 089 395
- US-A1- 2013 112 550

## Description

### BACKGROUND OF THE INVENTION

### 1. Field of the invention

The present invention relates to a gas generator for health use, more particularly, a anti-explosion gas generator for health use that can produce a combination gas of hydrogen and oxygen.

### 2. Description of the prior art

From ancient times till now, humanity has always made preserving life a high priority. Many developments in medical technology are used for diseases and increasing life expectancy. In the past, most medical treatment was passive. In other words, diseases are treated only when people fall ill, by performing surgical operation, medication, chemotherapy, radiation treatment and so on. But recently, many medical experts are focusing on disease prevention, such as studying on the health effects of food, and screening for genetic disorders to actively reduce the risk of falling ill. Furthermore, to increase life expectancy, many anti-aging technologies have been developed, including skin care products and antioxidant food/medicine.

In recent years, people have been noticing the benefits of aromatherapy. Aromatherapy is a natural way to make people feel relaxed and become healthy. Essential oils are extracted from aromatic plants to act as a medium which is then exposed to someone by massaging, bathing, perfuming and so on. This method has existed since the acient times of Egypt and is now gaining a lot of attention in Europe. A French scientist named René Maurice Gattefossé published his research results on aromatherapy in a scientific journal, which sparked interest in many people. His research found that the plant's essential oils can reach deep layer tissues of skin, which is then absorbed by blood vessels and to reach organs that need to be treated by blood circulation.

In the prior art, CN 202 576 577 U already disclosed a gas generator for generating gas mixture of hydrogen and oxygen for health use. However, the gas mixture of hydrogen and oxygen disclosed in CN 202 576 577 U will cause explosion in the gas generator easily, and it is insufficient for the health use. Therefore, the present invention provides a gas generator for health use which having anti-explosion function.

Other prior art devices are disclosed in US 2013/112550 A1, US 2010/089395 A1, EP 2 484 811 A1 and US 5 672 581 A.

Therefore, the present invention provides a gas generator for health use. The gas generator can produce health gas for health care that makes people feel relaxed and is also suitable for medical treatment.

### SUMMARY OF THE INVENTION

One objective of the present invention is to provide an anti-explosion gas generator for health use. The anti-explosion gas generator for health use can generate a gas mixture of hydrogen and oxygen for being inhaled by a user.

Another objective of the present invention is to provide an anti-explosion gas generator for health use. The anti-explosion gas generator for health use can generate a gas mixture of hydrogen and oxygen, then mix the gas mixture with an atomized medicine, water vapor or a volatile essential oil for being inhaled by a user.

The objectives are solved by an anti-explosion gas generator for health use according to claim 1

According to an example, the anti-explosion gas generator for health use comprises an electrolysis device and a gas mixing system. The electrolysis device is adapted for electrolyzing water to produce a gas mixture of hydrogen and oxygen. The gas mixing system is coupled to the electrolysis device to receive the gas mixture and to mix the gas mixture with water vapor, an atomized medicinal liquid, a volatile essential oil or a combination thereof, so as to produce a health gas for being inhaled by a user.

According to an example, the anti-explosion gas generator for health use further comprises a gas feeding element coupled between the electrolysis device and the gas mixing system. The gas feeding element is adapted for introducing a gas into the gas mixture of hydrogen and oxygen to reduce the concentration of hydrogen. Furthermore, in one of embodimnets the added gas is air, an inert gas, water vapor or a combinations thereof.

According to an example, the anti-explosion gas generator for health use further comprises a flow controller coupled to the electrolysis device. The flow controller is adapted for controlling the quantity of the gas mixture flowing into the gas mixing system, thereby reducing the concentration of hydrogen in the gas mixture.

According to an example, the anti-explosion gas generator for health use further comprises a flow meter coupled to the electrolysis device. When the flow meter senses an abnormal level of the gas mixture generated from the electrolysis device (such as sensing a gas level greater or less than a predetermined safety value), the flow meter will cut off the power supplied to the electrolysis device, thereby reducing the concentration of hydrogen in the gas mixture.

According to the present invention, the gas mixing system further comprises a humidifier and an atomized/volatile gas mixing tank. The humidifier is coupled to the electrolysis device for receiving the gas mixture to generate a filtered gas. The atomized/volatile gas mixing tank is coupled to the humidifier for receiving the filtered gas and then mixing the filtered gas with an atomized gas to generate a health gas.

According to another embodiment of the present invention, the anti-explosion gas generator for health use further comprises a conversion valve coupled between the humidifier and the atomized/volatile gas mixing tank. The conversion valve is adapted to selectively connect the humidifier to the atomized/volatile gas mixing tank, so that the filtered gas can be mixed with the atomized gas to generate the health gas. Further, the conversion valve can selectively disconnect the humidifier from the atomized/volatile gas mixing tank, so that the filtered gas is output directly. The humidifier comprises a pure water tank for filtering the gas mixture of hydrogen and oxygen to generate the filtered gas. The atomized/volatile gas mixing tank comprises an oscillator for atomizing or vaporizing a liquid to produce the atomized gas. The liquid can be an essential oil, a medicinal liquid, pure water or a combination thereof. The atomized gas can be a volatile essential oil, an atomized medicine, water vapor or a combination thereof. Furthermore, the anti-explosion gas generator for health use is configured to selectively output the filtered gas or the health gas by turning on/off the oscillator.

According to another embodiment of the present invention, the humidifier further comprises a pressure sensor and a release valve. The pressure sensor is used for detecting whether the pressure of the filtered gas excceds beyond a predetermied level. When the pressure is higher than the predetermined level, the pressure sensor is programmed to open the release valve to reduce the pressure of the filtered gas, thus achieving an explosion-proof effect.

The anti-explosion gas generator for health use according to the present invention can generate a gas mixture of hydrogen and oxygen, and the gas mixture can be selectively mixed with a volatile essential oil, an atomized medicine, water vapor or a combination thereof to form a health gas for being inhaled by a user. As the health gas includes a certain concentration of hydrogen, it provides anti-oxidation and an anti-aging effect. In addition, the atomized medicine in the health gas can be easily absorbed. Furthermore, the volatile essential oil in the health gas can relieve stress and improve the health of users.

Many other advantages and features of the present invention will be further understood by the following detailed description and the appended drawings.

### BRIEF DESCRIPTION OF THE APPENDED DRAWINGS

FIG. 1 is a schematic diagram illustrating an electrolysis device of an anti-explosion gas generator for health use;
FIG. 2 is a schematic diagram illustrating a gas mixing system of an anti-explosion gas generator for health use;
FIG. 3 is a schematic diagram illustrating a gas mixing system of an anti-explosion gas generator for health use.

To facilitate understanding, identical reference numerals have been used, where it is possible to designate identical elements that are common to the figures.

### DETAILED DESCRIPTION OF THE INVENTION

In order to allow the advantages and features of the present invention to be more easily and clearly understood, the embodiments and appended drawings thereof are discussed in the following. However, the present invention is not limited to the embodiments and appended drawings.

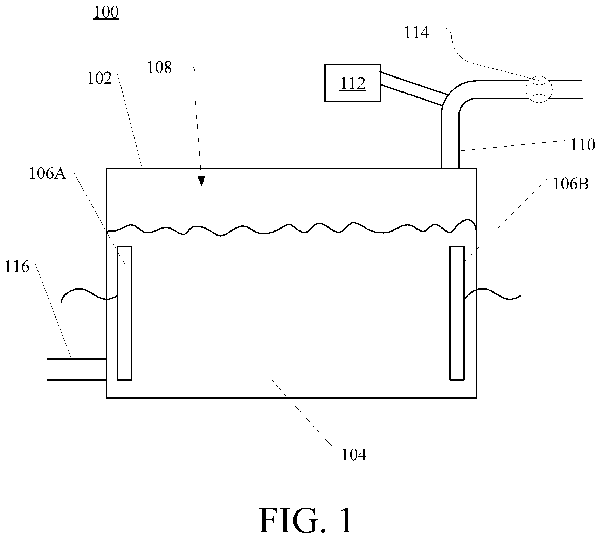

Please refer to FIG. 1. FIG. 1 is a schematic diagram illustrating an electrolysis device of an anti-explosion gas generator for health use. The present example provides an anti-explosion gas generator for health use which can generate a gas mixture of hydrogen and oxygen. In some embodiments, the gas mixture of hydrogen and oxygen is produced by the electrolysis of water. As shown in the figure, in some embodiments of the present invention, the electrolysis device 100 comprises an electrolysis tank 102 for accommodating electrolytic water 104. The main ingredients of the electrolytic water 104 is pure water, but electrolytes such as sodium hydroxide, calcium carbonate and sodium chloride can be added into the electrolyzed water 104 if necessary. The electrolysis tank 102 comprises two electrodes 106A and 106B, the two electrodes 106A and 106B respectively represent a cathode electrode and an anode electrode. The two electrodes 106A and 106B are coupled to a power supply (not shown) to provide the required power for the electrolysis device 100. In some embodiments, the polarity of the two electrodes 106A and 106B are fixed, for example, the electrode 106A is the cathode, the electrode 106B is the anode. In other embodiments, the polarity of the two electrodes 106A and 106B can be alternate, for example, at a point in time, the electrode 106A is the cathode and the electrode 106B is the anode, but after a predetermined time, the electrode 106A changes into the anode and the electrode 106B changes into the cathode.

After the two electrodes 106A and 106B are powered, the electrolyzed water 104 in the electrolysis tank 102 will start to be electrolyzed to generate hydrogen and oxygen. Hydrogen is formed on the cathode and oxygen is formed on the anode, and both hydrogen and oxygen are released to the upper part of the electrolysis tank 102 to form a gas mixture 108 of hydrogen and oxygen. The gas mixture 108 of hydrogen and oxygen is exported from a gas line 110 of the electrolysis tank 102 for usage. In another embodiment, hydrogen from the cathode and oxygen from the anode are exported and then mixed to form the gas mixture of hydrogen and oxygen. As the ratio of hydrogen to oxygen from the electrolysis of water is about 2:1 (H2: O2), the proportion of hydrogen in the combination gas may exceed 66%. In some embodiments, to prevent explosion of hydrogen, a gas feeding element 112 can be applied in the present invention to add a gas into the gas mixture 108, so as to reduce the concentration of hydrogen to an amount, for example, between 2% to 60%, such as an amount between 2% to 4%., wherein the gas can be air, water vapor, an inert gas (such as nitorgen), oxygen or combination thereof.

Reducing the flow rate of the gas mixture 108 can also have an explosion-proof effect. Therefore, in another embodiment, the gas line 110 comprises a flow controller 114 for controlling the flow rate of the gas mixture 108, so that the concentration of hydrogen in the gas mixture 108 can be reduced when the gas mixture 108 is transferred to a downstream device and mixed with the gas in the downstream device. In one embodiment, the flow controller 114 comprises a flow meter for detecting whether the flow rate of the gas mixture 108 from the electrolysis tank 102 is greater than a dangerous level (for example, the dangerous value should not exceed two liters per minute, i.e., 2L/min, or 2000c.c/min). The flow controller 114 can selectively shut down the power supplied to the electrolysis tank 102 to prevent excessive concentration of hydrogen. Sometimes the instability of the power supply will cause the concentration of hydrogen to be too high, thus, the gas line 110 and the electrolysis tank 102 can also be provided with the flow meter. However, if the concentration of hydrogen is too low, the health benefits of the present invention will be diminished. Therefore, the above dangerous level is preferably set to be no less than 0.1L/min and more preferably between 0.1L/min and 0.2L/min. Of course, the flow meter (or the flow controller 114) can also be mounted at other locations as long as the flow rate of the gas mixture can be detected precisely. In other embodiments, the flow controller can comprise the flow meter and a computer (not shown), and the computer is stored with a pre-set parameter (such as reference table) representing the relationship between the power level (voltage x current) of the power supply and the flow rate of the gas mixture of hydrogen and oxygen. The computer is used for calculating parameter values every fixed time interval (for example, estimating the power level from the detected flow rate according to the reference table). If the flow rate of the gas mixture fails to correspond to the power level, it might indicate that the pressure of the gas mixture is too high and the flow controller would be activated to adjust the power supplied to the electrolysis tank 102, thereby reducing the produced amount of the gas mixture 108. That is to say, the flow controller 114 can selectively adjust the power level supplied to the electrolysis tank 102. Therefore, with the adjustment of the power level, the flow controller can control the flow rate of the gas mixture 108 to be between 0.1L/min and 2L/min and, more preferably, between 0.1L/min and 0.2L/min.

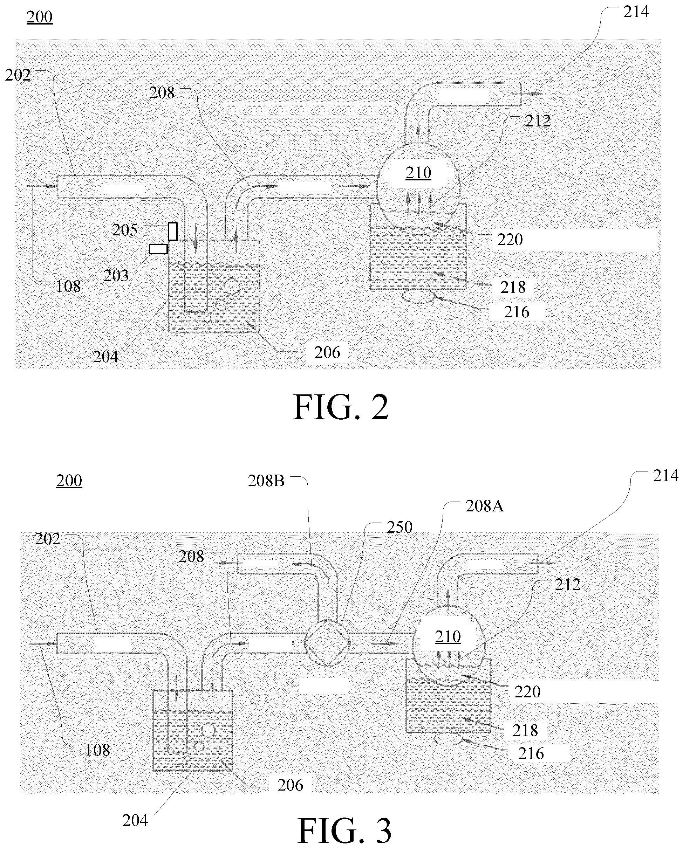

Please refer to FIG. 2. FIG. 2 is a schematic diagram illustrating a gas mixing system of an anti-explosion gas generator for health use according to the invention. The gas mixing system 200 is coupled to the electrolysis device 100 in FIG. 1, such as via the gas line 110 shown in FIG. 1, to receive the gas mixture 108. The gas mixing system 200 comprises a humidifier 204, such as a sink or a pure water tank, for filtering the gas mixture 108 with pure water 206, thereby generating the filtered gas 208. In some embodiments, the humidifier 204 is adpated to filter out other gases or impurities in the gas mixture 108 except hydrogen and oxygen, such as chlorine or a trace mount of metals. Therefore, the humidifier 204 is not limited to the pure water tank in this embodiment and can be any type of apparatus which is adapted to absorb gases other than hydrogen and oxygen. The gas mixing system 200 further comprises an atomized/volatile gas mixing tank 210 coupled to the humidifier 204 for receiving the filtered gas 208. The filtered gas 208 is then mixed with an atomized gas 212 to form a health gas 214. The atomized/volatile gas mixing tank 210 further comprises an oscillator 216 for atomizing or vaporizing a liquid 218 and a liquid 220 in the atomized/volatile gas mixing tank 210, thereby generating the atomized gas 212. The liquid 218 can be pure water to serve as an atomized base liquid. The liquid 220 can be an essential oil, a medicinal liquid, pure water or a combination thereof. The atomized gas 212 can be a volatile essential oil, an atomized medicine, water vapor or a combination thereof.

Furthermore, the anti-explosion gas generator for health use further comprises a pressure sensor 203 and a release valve 205 which is electrically connected to the pressure sensor 203. For example, as shown in FIG. 2, the pressure sensor 203 and the release valve 205 are coupled to the humidifier 204, wherein the pressure sensor 203 is used for detecting whether the pressure of the filtered gas is greater than a dangerous level (For example, 1 atm, i.e., 1Pa). If the pressure of the filtered gas is too high, the pressure sensor 203 would open the release valve 205 to reduce the pressure of the filtered gas, thus achieving an explosion-proof effect. In other words, the pressure sensor 203 can selectively open the release valve 205. Of course, the pressure sensor 203 and the release valve 205 can be coupled to other locations as long as the pressure sensor 203 and the release valve 205 can detect and reduce the pressure of the gas mixture. For example, the pressure sensor 203 and the release valve 205 can be coupled to the electrolysis device 100. By using the gas feeding element 112, the flow controller 114 (or the flow meter), the pressure sensor 203 and the release valve 205 in combination, the present invention can reduce the concentration of hydrogen and have an explosion-proof effect.

In other embodiments, the flow controller comprise the flow sensor and a computer (not shown), where the computer is stored with a pre-set parameter (such as a reference table) representing the relationship between the power level (voltage x current) of the power supply and the flow rate of the gas mixture of hydrogen and oxygen. The computer is adapted for calculating parameter values every fixed time interval (for example, estimating the power level from the detected flow rate according to the reference table). If the flow rate of the gas mixture fails to correspond to the power level, it might indicate that the pressure of the gas mixture is too high, and the computer would open the release valve 205 until the pressure reaches a preset safety value, and then close the release valve 205. However, if the release valve is opened too many times during a period of time (which can be cumulatively calculated by a counter) or there is a gas leakage problem, the computer can cut off the power of the electrolysis tank for security. That is to say, the computer can selectively open the release valve and cut off the power of the electrolysis tank.

Please refer to FIG. 3. FIG. 3 is a schematic diagram illustrating a gas mixing system of an anti-explosion gas generator for health use according to another embodiment of the invention. In this embodiment, the anti-explosion gas generator for health use further comprises a conversion valve 250 coupled between the humidifier 204 and the atomized/volatile gas mixing tank 210, wherein the conversion valve 205 is adapted to selectively connect the humidifier 204 to the atomized/volatile gas mixing tank 210, so that the atomized gas 212 can be mixed with the filtered gas 208A to generate the health gas 214. The conversion valve is also adapted to selectively disconnect the humidifier from the atomized/volatile gas mixing tank, so that the filtered gas is directly output for being inhaled by a user. That is to say, the user has a choice in deciding whether the health gas should include the volatile essential oil, atomized medicine or water vapor or not by switching the conversion valve. This embodiment differs the embodiment of FIG. 2 in that this embodiment allows the user to breathe a gas mixture comprising only hydrogen and oxygen.

In another embodiment, a user can decide the composition of the health gas by other ways, such as by turning on/off the oscillator 216. For example, if the oscillator 216 is turned on, the filtered gas 208 will be mixed with the atomized gas 212 to generate the health gas 214; if the oscillator 216 is turned off, the filtered gas 208 will be exported directly for being inhaled by a user.

According to the above embodiments, the health gas 214 comprises hydrogen and oxygen, and optionally a volatile essential oil, an atomized medicine, water vapor or a combination thereof. Studies have found that there is an instable oxygen species (O+), also known as free radicals, in the human body. The free radicals are usually generated due to diseases, diet, environment and one's life style, and the free radicals in the human body can be excreted in the form of water by reacting with the inhaled hydrogen. With this not claimed method, the amount of free radicals in the human body can be reduced, thereby restoring the body condition from an acidic state to an alkaline state, achieving an anti-oxidation, anti-aging and beauty health effect, and even eliminating chronic diseases. In addition, according to the clinical studies, the atomized medicinal liquid is easier absorbed by the human body than its non-atomized counterpart. That is to say, compared with its non-atomized counterpart, the atomized medicine can achieve the same therapeutic effect with a much lower dosage amount. Furthermore, the drug's side effects can be reduced due to the low dosage amount of the atomized medicine administered. Therefore, the health gas 214 may lead to an excellent therapeutic effect. There are also clinical experiments showing that, for patients who need to inhale a high concentration of oxygen for a long time, the lung damage from the high concentration of oxygen can be ameliorated by inhaling hydrogen. Aside from those benefits, the volatile essential oil in the health gas can help general users improve their health and relieve stress.

## Claims

1. An anti-explosion gas generator for health use comprising an electrolysis device (100) for electrolyzing water to produce a gas comprising hydrogen, comprising:
a power supply electrically connected to the electrolysis device (100) and adapted for supplying power to the electrolysis device;
a flow controller (114) configured to control the flow rate of the gas comprising hydrogen, wherein the flow controller selectively adjusts the power level of the power supply so that the flow rate of the gas comprising hydrogen is controllable more than 0.1L/min;
**characterized by** further comprising:
a humidifier (204) coupled to the electrolysis device (100) to filter the gas comprising hydrogen; and
an atomized/volatile gas mixing tank (210) coupled to the humidifier (204), the atomized/volatile gas mixing tank (210) being configured to receive the filtered gas, to generate an atomized gas and to mix the atomized gas with the filtered gas comprising hydrogen, wherein the atomized gas is a water vapor, an atomized medicine, a volatile essential oil, or a combination thereof.

2. The anti-explosion gas generator for health use of claim 1 further comprising a conversion valve (250) coupled between the humidifier (204) and the atomized/volatile gas mixing tank (210), wherein the conversion valve is adapted to selectively connect the humidifier (204) to the atomized/volatile gas mixing tank (210) so that the atomized gas is mixed with the filtered gas, and wherein the conversion valve is adapted to selectively disconnect the humidifier (204) from the atomized/volatile gas mixing tank so that the gas comprising hydrogen is output from the anti-explosion gas generator.

3. The anti-explosion gas generator for health use of claim 1, wherein the atomized/volatile gas mixing tank (210) is configured to selectively generate the atomized gas, and the atomized/volatile gas mixing tank (210) is outputted the gas comprising hydrogen when the atomized/volatile gas mixing tank (210) does not generate the atomized gas, or outputted a mixed gas comprising the atomized gas and the gas comprising hydrogen when the atomized/volatile gas mixing tank (210) generates the atomized gas.

4. The anti-explosion gas generator for health use of claim 3, wherein the atomized/volatile gas mixing tank (210) further comprises an oscillator (216) for atomizing or vaporizing a liquid to produce the atomized gas, and the anti-explosion gas generator for health use is adapted to selectively output either the gas comprising hydrogen or a gas comprising the atomized gas and the gas comprising hydrogen by turning on/off the oscillator.

5. The anti-explosion gas generator for health use of claim 1 further comprising a gas feeding element (112) coupled to the electrolysis device and adapted for introducing a gas into the gas comprising hydrogen to reduce the concentration of hydrogen, wherein the gas is air, water vapor, an inert gas, oxygen or a combination thereof.

6. The anti-explosion gas generator for health use of claim 1, further comprises a flow meter adapted for detecting the flow rate of the gas comprising hydrogen and configured to selectively shut down the electrical connection between the electrolysis device (100) and the power supply.

7. The anti-explosion gas generator for health use of claim 6, further comprises a computer and a release valve (205), wherein the computer is coupled to the flow meter and stored with a reference table representing the relationship between the power level of the power supply and the flow rate of the gas comprising hydrogen, and the computer is configured to selectively open the release valve.

8. The anti-explosion gas generator for health use of claim 1, further comprising a release valve (205) and a pressure sensor (203), wherein the pressure sensor (203) is adapted for detecting whether the gas pressure of the anti-explosion gas generator is greater than a dangerous level and selectively open the release valve to reduce the gas pressure.

9. The anti-explosion gas generator for health use of claim 1, further comprising a pressure sensor (203) adapted for detecting whether the gas pressure of the anti-explosion gas generator is greater than a dangerous level and configured to selectively shut down the electrical connection between the electrolysis device (100) and the power supply.

## Patentansprüche

1. Explosionsgeschützter Gasgenerator zur Gesundheitsverwendung mit einer Elektrolysevorrichtung (100) zur Elektrolyse von Wasser, um ein wasserstoffhaltiges Gas zu erzeugen, mit:
einer Leistungsversorgung, die mit der Elektrolysevorrichtung (100) elektrisch verbunden und angepasst ist, die Elektrolysevorrichtung mit Leistung zu versorgen;
einer Durchflusssteuerungseinrichtung (114), die konfiguriert ist, den Durchfluss des wasserstoffhaltigen Gases zu steuern, wobei die Durchflusssteuerungseinrichtung das Leistungsniveau der Leistungsversorgung selektiv einstellt, sodass der Durchfluss des wasserstoffhaltigen Gases auf mehr als 0,1 l/min steuerbar ist;
**dadurch gekennzeichnet, dass** er ferner aufweist:
eine Befeuchtungseinrichtung (204), die mit der Elektrolysevorrichtung (100) gekoppelt ist, um das wasserstoffhaltige Gas zu filtern; und
einen Mischbehälter (210) für zerstäubtes/flüchtiges Gas, der mit der Befeuchtungseinrichtung (204) gekoppelt ist, wobei der Mischbehälter (210) für zerstäubtes/flüchtiges Gas konfiguriert ist, das gefilterte Gas aufzunehmen, um ein zerstäubtes Gas zu erzeugen, und das zerstäubte Gas mit dem gefilterten wasserstoffhaltigen Gas zu vermischen, wobei das zerstäubte Gas ein Wasserdampf, ein zerstäubtes Medikament, ein flüchtiges ätherisches Öl oder eine Kombination davon ist.

2. Explosionsgeschützter Gasgenerator zur Gesundheitsverwendung nach Anspruch 1, ferner mit einem Umstellventil (250), das zwischen der Befeuchtungseinrichtung (204) und dem Mischbehälter (210) für zerstäubtes/flüchtiges Gas gekoppelt ist, wobei das Umstellventil angepasst ist, die Befeuchtungseinrichtung (204) mit dem Mischbehälter (210) für zerstäubtes/flüchtiges Gas selektiv zu verbinden, sodass das zerstäubte Gas mit dem gefilterten Gas vermischt wird, und wobei das Umstellventil angepasst ist, die Befeuchtungseinrichtung (204) von dem Mischbehälter für zerstäubtes/flüchtiges Gas selektiv zu trennen, sodass das wasserstoffhaltige Gas aus dem explosionsgeschützten Gasgenerator ausgegeben wird.

3. Explosionsgeschützter Gasgenerator zur Gesundheitsverwendung nach Anspruch 1, wobei der Mischbehälter (210) für zerstäubtes/flüchtiges Gas konfiguriert ist, das zerstäubte Gas selektiv zu erzeugen, und der Mischbehälter (210) für zerstäubtes/flüchtiges Gas das wasserstoffhaltige Gas ausgibt, wenn der Mischbehälter (210) für zerstäubtes/flüchtiges Gas das zerstäubte Gas nicht erzeugt, oder ein Mischgas, das das zerstäubte Gas und das wasserstoffhaltige Gas enthält, ausgibt, wenn der Mischbehälter (210) für zerstäubtes/flüchtiges Gas das zerstäubte Gas erzeugt.

4. Explosionsgeschützter Gasgenerator zur Gesundheitsverwendung nach Anspruch 3, wobei der Mischbehälter (210) für zerstäubtes/flüchtiges Gas ferner einen Oszillator (216) zum Zerstäuben oder Verdampfen einer Flüssigkeit aufweist, um das zerstäubte Gases zu erzeugen, und der explosionsgeschützte Gasgenerator zur Gesundheitsverwendung angepasst ist, entweder das wasserstoffhaltige Gas oder ein Gas, das das zerstäubte Gas und das wasserstoffhaltige Gas enthält, durch Ein-/Ausschalten des Oszillators selektiv auszugeben.

5. Explosionsgeschützter Gasgenerator zur Gesundheitsverwendung nach Anspruch 1, ferner mit einem Gaszuführelement (112), das mit der Elektrolysevorrichtung gekoppelt und angepasst ist, ein Gas in das wasserstoffhaltige Gas einzuleiten, um die Konzentration von Wasserstoff zu reduzieren, wobei das Gas Luft, Wasserdampf, ein inertes Gas, Sauerstoff oder eine Kombination davon ist.

6. Explosionsgeschützter Gasgenerator zur Gesundheitsverwendung nach Anspruch 1, ferner mit einer Durchflussmesseinrichtung, die zum Erfassen des Durchflusses des wasserstoffhaltigen Gases angepasst und konfiguriert ist, die elektrische Verbindung zwischen der Elektrolysevorrichtung (100) und der Leistungsversorgung selektiv abzuschalten.

7. Explosionsgeschützter Gasgenerator zur Gesundheitsverwendung nach Anspruch 6, ferner mit einem Computer und einem Ablassventil (205), wobei der Computer mit der Durchflussmesseinrichtung gekoppelt ist und eine Referenztabelle speichert, die die Beziehung zwischen dem Leistungsniveau der Leistungsversorgung und dem Durchfluss des wasserstoffhaltigen Gases darstellt, und der Computer konfiguriert ist, das Ablassventil selektiv zu öffnen.

8. Explosionsgeschützter Gasgenerator zur Gesundheitsverwendung nach Anspruch 1, ferner mit einem Ablassventil (205) und einem Drucksensor (203), wobei der Drucksensor (203) angepasst ist, zu erfassen, ob der Gasdruck des explosionsgeschützten Gasgenerators größer als ein gefährliches Niveau ist, und das Ablassventil selektiv zu öffnen, um den Gasdruck zu reduzieren.

9. Explosionsgeschützter Gasgenerator zur Gesundheitsverwendung nach Anspruch 1, ferner mit einem Drucksensor (203), der angepasst ist, zu erfassen, ob der Gasdruck des explosionsgeschützten Gasgenerators größer als ein gefährliches Niveau ist, und der konfiguriert ist, die elektrische Verbindung zwischen der Elektrolysevorrichtung (100) und der Leistungsversorgung selektiv abzuschalten.

## Revendications

1. Générateur de gaz anti-explosion pour usage médical, comprenant un dispositif d'électrolyse (100) pour électrolyser de l'eau afin de produire un gaz comprenant de l'hydrogène, comprenant :
une alimentation électrique connectée électriquement au dispositif d'électrolyse (100), adaptée pour alimenter en énergie le dispositif d'électrolyse ;
un régulateur de débit (114) configuré pour réguler le débit du gaz comprenant de l'hydrogène, lequel régulateur de débit ajuste le niveau d'énergie de l'alimentation électrique de façon que le débit du gaz comprenant de l'hydrogène puisse être régulé à plus de 0,1 l/min ;
**caractérisé en ce qu'**il comprend en outre :
un humidificateur (204) couplé au dispositif d'électrolyse (100) pour filtrer le gaz comprenant de l'hydrogène ; et
un réservoir de mélange de gaz atomisé/volatil (210) couplé à l'humidificateur (204), le réservoir de mélange de gaz atomisé/volatil (210) étant configuré pour recevoir le gaz filtré, pour générer un gaz atomisé et pour mélanger le gaz atomisé avec le gaz filtré comprenant de l'hydrogène, dans lequel le gaz atomisé est de la vapeur d'eau, un médicament atomisé, une huile essentielle volatile ou une combinaison de ceux-ci.

2. Générateur de gaz anti-explosion pour usage médical selon la revendication 1, comprenant en outre une soupape de conversion (250) couplée entre l'humidificateur (204) et le réservoir de mélange de gaz atomisé/volatil (210), dans lequel la soupape de conversion est adaptée pour connecter sélectivement l'humidificateur (204) au réservoir de mélange de gaz atomisé/volatil (210) de sorte que le gaz atomisé soit mélangé avec le gaz filtré, et dans lequel la soupape de conversion est adaptée pour déconnecter sélectivement l'humidificateur (204) du réservoir de mélange de gaz atomisé/volatil de sorte que le gaz comprenant de l'hydrogène soit délivré en sortie depuis le générateur de gaz anti-explosion.

3. Générateur de gaz anti-explosion pour usage médical selon la revendication 1, dans lequel le réservoir de mélange de gaz atomisé/volatil (210) est configuré pour générer sélectivement le gaz atomisé, et le réservoir de mélange de gaz atomisé/volatil (210) délivre en sortie le gaz comprenant de l'hydrogène quand le réservoir de mélange de gaz atomisé/ volatil (210) ne génère pas de gaz atomisé, ou délivre en sortie un mélange gazeux comprenant le gaz atomisé et le gaz comprenant de l'hydrogène quand le réservoir de mélange de gaz atomisé/volatil (210) génère le gaz atomisé.

4. Générateur de gaz anti-explosion pour usage médical selon la revendication 3, dans lequel le réservoir de mélange de gaz atomisé/volatil (210) comprend en outre un oscillateur (216) pour atomiser ou vaporiser un liquide de façon à produire le gaz atomisé, et le générateur de gaz anti-explosion pour usage médical est adapté pour délivrer en sortie soit le gaz comprenant de l'hydrogène soit un gaz comprenant le gaz atomisé et le gaz comprenant de l'hydrogène par mise sous tension/hors tension de l'oscillateur.

5. Générateur de gaz anti-explosion pour usage médical selon la revendication 1, comprenant en outre un élément d'alimentation en gaz (112) couplé au dispositif d'électrolyse et adapté pour introduire un gaz dans le gaz comprenant de l'hydrogène pour réduire la concentration d'hydrogène, dans lequel le gaz est de l'air, de la vapeur d'eau, un gaz inerte, de l'oxygène ou une combinaison de ceux-ci.

6. Générateur de gaz anti-explosion pour usage médical selon la revendication 1, comprenant en outre un débitmètre adapté pour détecter le débit du gaz comprenant de l'hydrogène et configuré pour couper sélectivement la connexion électrique entre le dispositif d'électrolyse (100) et l'alimentation électrique.

7. Générateur de gaz anti-explosion pour usage médical selon la revendication 6, comprenant en outre un ordinateur et une vanne de détente (205), dans lequel l'ordinateur est couplé au débitmètre et stocke en mémoire une table de référence représentant la relation entre le niveau d'énergie de l'alimentation électrique et le débit du gaz comprenant de l'hydrogène, et l'ordinateur est configuré pour ouvrir sélectivement la vanne de détente.

8. Générateur de gaz anti-explosion pour usage médical selon la revendication 1, comprenant en outre une vanne de détente (205) et un capteur de pression (203), dans lequel le capteur de pression (203) est adapté pour détecter si la pression de gaz du générateur de gaz anti-explosion est supérieure à un niveau dangereux et ouvrir sélectivement la vanne de détente pour réduire la pression de gaz.

9. Générateur de gaz anti-explosion pour usage médical selon la revendication 1, comprenant en outre un capteur de pression (203) adapté pour détecter si la pression de gaz du générateur de gaz anti-explosion est supérieure à un niveau dangereux et configuré pour couper sélectivement la connexion électrique entre le dispositif d'électrolyse (100) et l'alimentation électrique.
